(19) Europäisches Patentamt / European Patent Office / Office européen des brevets

(11) **EP 4 097 827 B1**

(12) **EUROPÄISCHE PATENTSCHRIFT**

(45) Veröffentlichungstag und Bekanntmachung des Hinweises auf die Patenterteilung:
**09.04.2025   Patentblatt 2025/15**

(21) Anmeldenummer: **21701528.8**

(22) Anmeldetag: **22.01.2021**

(51) Internationale Patentklassifikation (IPC):
*H02K 3/28* (2006.01)   *H02K 11/28* (2016.01)
*A61B 17/00* (2006.01)

(52) Gemeinsame Patentklassifikation (CPC):
**H02K 3/28; H02K 11/28;** A61B 2017/00398;
A61B 2017/00477; A61B 2017/00734;
H02K 2213/09

(86) Internationale Anmeldenummer:
**PCT/EP2021/051487**

(87) Internationale Veröffentlichungsnummer:
**WO 2021/151798 (05.08.2021 Gazette 2021/31)**

(54) **MULTI BETRIEBSSPANNUNGSMOTOR**

MULTI-OPERATING-VOLTAGE MOTOR

MOTEUR À TENSIONS DE FONCTIONNEMENT MULTIPLES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität:  **28.01.2020   DE 102020101990**

(43) Veröffentlichungstag der Anmeldung:
**07.12.2022   Patentblatt 2022/49**

(73) Patentinhaber: **Aesculap AG**
**78532 Tuttlingen (DE)**

(72) Erfinder:
• **HÖGERLE, Roland-Alois**
**78532 Tuttlingen (DE)**
• **BARTH, Jürgen**
**78588 Denkingen (DE)**

(74) Vertreter: **Winter, Brandl - Partnerschaft mbB**
**Alois-Steinecker-Straße 22**
**85354 Freising (DE)**

(56) Entgegenhaltungen:

| | |
|---|---|
| EP-A1- 3 435 540 | EP-A1- 3 534 532 |
| EP-A2- 1 450 469 | WO-A1-2013/155601 |
| WO-A1-2018/213919 | JP-A- 2017 121 158 |
| US-A1- 2018 256 287 | |

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

### Technisches Gebiet

Die vorliegende Offenbarung betrifft einen elektrischen Motor mit einer Motorwicklungsverschaltung für das Betreiben eines elektrochirurgischen Instruments mit genau einer Motorplattform.

### Technischer Hintergrund

Elektrische Motorensysteme für die Chirurgie können in zwei Antriebsklassen bzw. Betriebsspannungsklassen eingeteilt werden. In der einen der beiden Antriebsklassen bzw. Betriebsspannungsklassen werden die Systeme mit einem Akku betrieben und in der anderen der beiden Antriebsklassen bzw. Betriebsspannungsklassen weisen die Systeme einen Netzanschluss auf, welcher an ein (Strom-)Netz angeschlossen wird, um den elektrischen Motor und somit daran angeschlossene/angesteckte elektrochirurgische Instrument. Hierbei wird ein mit einem Akku betriebenes System mit einer Spannung von 9,6 Volt bis 14 Volt versorgt und ein mit einem Netzanschluss bzw. einem (Strom-)Netz betriebenes System wird mit einer Spannung von 36 Volt bis 48 Volt versorgt.

Es ist Stand der Technik bekannt, in welchem für ein akkubetriebenes System eine eigene Motorplattform mit etwa 12 Volt vorgesehen ist und für ein netzbetriebenes System eine eigene Motorplattform mit etwa 36 Volt vorgesehen ist. Durch das Verwenden von zwei verschiedenen Motorplattform ist nicht gegeben, das gleiche chirurgische Instrument flexibel mit einem Akku oder einem Netzanschluss zu betreiben.

Aus verschiedenen Gründen gilt es die beiden Betriebsspannungsklassen auf einer Motorplattform zu vereinen. Dies bringt zum einen fertigungstechnische, kostentechnische und zulassungstechnische und zum anderen anwendertechnische Vorteile mit sich.

In dem bereits bekannten Stand der Technik, hat das Vereinen der beiden Motorplattformen auf eine einheitliche Motorplattform jedoch mehrere Nachteile. In einem ersten Fall, in dem das Betreiben des Systems mit einem Akkuantrieb auf 36 Volt erhöht wird, wäre die Folge ein hohes Maschinengewicht sowie ein hohes Maschinenvolumen. Dies ist für den Anwender inakzeptabel, da es ein präzises Arbeiten und ein Arbeiten über einen längeren Zeitraum erheblich erschwert.

In einem zweiten Fall, in welchem man das Betreiben des Systems mit einem Netzanschluss auf 12 Volt reduziert, wäre die Folge eine sehr ungünstige Motorauslegung. Insbesondere ein High-Speed Antrieb, also ein Antrieb mit hoher Geschwindigkeit von beispielsweise mehr als 80.000 Umdrehungen pro Minute, wäre in dem zweiten Fall aufgrund der mit einhergehenden hohen Wärmeentwicklung ebenfalls für den Anwender inakzeptabel.

Des Weiteren ist aus dem Stand der Technik lediglich eine Stern-Dreieck-Umschaltung bekannt. Hier wird jedoch nur erreicht, dass bei gleicher Betriebsspannung der Motor in zwei unterschiedlichen Drehzahl- und Leistungsbereichen betrieben werden kann.

Somit betrifft beispielsweise die DE 10 2013 009 036 A1 eine Antriebseinheit für eine Zerkleinerungsvorrichtung, welche einen Stern-/Dreieck-umschaltbaren Motor, einen Antriebsstromrichter zur Regelung oder Steuerung einer physikalischen Größe des Motors und eine Steuereinheit aufweist, wobei Schaltvorrichtungen in der Antriebseinheit vorgesehen sind, welche die Stern-/Dreieck-Umschaltung ermöglichen, wobei die Auswahl der Stellung der Schaltvorrichtungen durch die Steuereinheit erfolgt und wobei die Steuereinheit dafür mindestens eine in der Antriebseinheit erfasste Größe berücksichtigt.

In der EP 3 400 645 A1 ist eine Umschalteinrichtung beschrieben, die mindestens einen elektrischen Schalter aufweist und dazu ausgebildet ist, die Motorwindungen in Abhängigkeit von der Schaltstellung als Stern oder als Dreieck zu verschalten.

Die JP 2017 121158 A betrifft einen Motor und ein Elektrowerkzeug, die in der Lage sind, eine Differenz der Widerstandswerte einer Vielzahl von Spulen, die parallel miteinander verbunden sind, zu unterdrücken, während die Verwendung eines großen Motorhauptkörpers vermieden wird.

Die EP 3 435 540 A1 betrifft eine Vorrichtung zum Modifizieren einer elektrischen Konfiguration eines Stators eines Wechselstrommotors.

Die US 2018/256 287 A1 betrifft ein Werkzeugerkennungssystem, welches ein für einen chirurgischen Eingriff ausgewähltes Werkzeug erkennt.

Die WO 2013/155 601 A1 betrifft eine elektrische Maschine, bei welcher der Stator Schalter umfasst, die es ermöglichen, die Spulen verschiedener Phasen in einer Sternkonfiguration, einer Dreieckkonfiguration oder einer neutralen Konfiguration miteinander zu verbinden.

Die EP 1 450 469 A2 betrifft einen Elektromotor, der in der Lage ist, leicht eine erforderliche Ausgangscharakteristik zu erreichen.

Die WO 2018/213 919 A1 betrifft eine elektrische Maschine mit Spulen oder Wicklungen, als dynamische Reaktion auf Betriebsbedingungen und unter Last.

Die EP 3 534 532 A1 betrifft eine Klimaanlage mit einer Signalempfangseinheit zum Empfangen eines Betriebsbefehlssignals, einem Rotationskompressor mit einem Motor mit Spulen, und einem mit den Spulen verbundenen Inverter.

Des Weiteren ist aus dem Stand der Technik der Dahlandermotor bekannt. Hier wird ebenfalls durch Umschalten der Wicklung von "Dreieck- in Doppelstern" erreicht, dass der Motor bei gleicher Betriebsspannung in zwei unterschiedlichen Drehzahl- und Leistungsbereichen betrieben werden kann. Das heißt, der Dahlandermotor ist eine Schaltungsvariante zum Umschalten zwischen verschiedenen Drehzahlen.

Daher werden Stand heute zwei eigenständige, unterschiedliche Motorplattformen angeboten. Im Vergleich zu einer einheitlichen Motorplattform ist dies nachteilig aufgrund eines höheren Fertigungsaufwands, Entwicklungsaufwands, Zulassungsaufwand und höheren Kosten, sowie eine schlechtere Gebrauchstauglichkeit beim Anwender und einem höheren Aufwand bei der Motordatenerfassung, Motordatenauswertung sowie der Integration ins IomT ("Internet of medical things", das heißt, medizinische Geräte und Tools, die über das Internet mit entsprechenden IT-Systemen verbunden sind).

Darüber hinaus können die aus dem Stand der Technik bekannten Varianten lediglich die Betriebsspannungsverhältnisse, also das Verhältnis zwischen einer akkubetriebenen Betriebsspannung zu deiner netzbetriebenen Betriebsspannung nach einem Umschalten zwischen den beiden Betriebsmöglichkeiten, 1:2 oder √3 bedienen.

Demnach liegt der vorliegenden Erfindung die Aufgabe zugrunde, die aus dem Stand der Technik bekannten Probleme zu beheben oder zumindest zu mindern. Insbesondere liegt der vorliegenden Erfindung die Aufgabe zugrunde, einen elektrischen Motor mit einer einheitlichen Motorplattform anzubieten, der sowohl im Akkubetrieb als auch im Netzbetrieb gleichmäßig und ideal läuft.

In anderen Worten, gilt es einen elektrischen Motor anzubieten, der mit beiden Betriebsspannungen gleichermaßen ideal ausgelegt ist und daher gleichermaßen läuft. Die Motorcharakteristik soll mit beiden Betriebsspannungen identisch sein, damit eine gemeinsame Zulassungsstrategie für beide Betriebsspannungsklassen erfolgen kann. Die Abmaße sowie die Handhabung der Motoren durch Nutzer bzw. Anwender sollen dabei keine negativen Auswirkungen zeigen.

Diese Aufgabe wird durch eine Motorwicklungsverschaltung eines elektrischen Motors zum Betreiben eines elektrochirurgischen Instruments gemäß Anspruch 1 gelöst.

Vorteilhafte Weiterbildungen der Erfindung sind Gegenstand der Unteransprüche.

**Zusammenfassung der Erfindung**

Ein elektrischer Motor mit einer Motorwicklungsverschaltung für das Betreiben eines elektrochirurgischen Instruments hat genau eine Motorplattform. Diese Motorplattform ist dazu vorgesehen und ausgebildet, den Motor in einer ersten Betriebsspannung und einer zur ersten Betriebsspannung unterschiedlichen zweiten Betriebsspannung zu betreiben. Zudem ist auf der Motorplattform eine Schaltung vorgesehen, vorzugsweise eine Dreiecksschaltung oder Sternschaltung, die jeweils durch drei Phasen mit jeweils einer Phasenspule ausgebildet ist, und dabei ist zumindest eine Phasenspule aus N>1 miteinander verschalteten Einzelspulen ausgebildet.

Die vorstehende Aufgabe wird dadurch gelöst, dass eine Motorwicklungsverschaltung gemäß dem vorstehenden Aspekt in der ersten Betriebsspannung die N Einzelspulen jeder Phasenspule parallel und in der zweiten Betriebsspannung die N Einzelspulen jeder Phasenspule in Reihe geschaltet sind und das Umschalten zwischen den beiden Betriebsspannungen mittels zumindest eines Kurzschlussmittels je Phasenspule vorgesehen ist.

In anderen Worten bedeutet das, durch einfaches Kurzschließen unterschiedlicher Potentiale den Motor bei unterschiedlichen Betriebsspannungen und dabei gleichbleibender Motorcharakteristik und gleichen Kennwerten zu betreiben. Demnach ist es vorgesehen, die Verschaltung der N Einzelspulen derart mit Hilfe zumindest eines Kurzschlussmittels von einer Reihenschaltung in eine Parallelschaltung bzw. von einer Parallelschaltung in eine Reihenschaltung umzuschalten, um die entsprechend erforderliche erste oder zweite Betriebsspannung für den Akkubetrieb oder den Netzbetrieb zu erhalten.

Dies hat den Vorteil, dass die Motorplattform für beide Betriebssysteme - Akkubetrieb und Netzbetrieb - identisch ausgebildet ist und keine kritischen Elemente wie Schalter, elektronische Bauteile oder dergleichen erforderlich sind, um zwischen dem Akkubetrieb und dem Netzbetrieb bzw. dem Netzbetrieb und dem Akkubetrieb umzuschalten. Zudem ist es vorteilhaft, dass die vorliegende Erfindung sowohl bei einer Sternschaltung als auch einer Dreiecksschaltung gleichermaßen funktioniert.

Eine beispielhafte Ausführungsform kann in dem akkubetriebenen System beispielsweise im Dreieck verschaltet sein, mit einem Drahtdurchmesser von vorzugsweise 0,355mm. In diesem Fall besteht jede Phasenspule aus drei parallel geschalteten Einzelspulen mit je dreizehn Windungen (insgesamt 39 Windungen je Phasenspule) und gibt 12 Volt aus. In dem dazu entsprechenden netzbetriebenen System ist eine beispielhafte Ausführungsform ebenfalls im Dreieck verschaltet mit einem Drahtdurchmesser von vorzugsweise 0,355mm und drei Einzelspulen mit jeweils dreizehn Windungen (insgesamt 39 Windungen pro Phasenspule) für jede Phasenspule, welche jeweils in Reihe geschaltet sind und 36 Volt ausgeben.

Es ist bevorzugt, wenn jede Phasenspule N Einzelspulen hat und die erste Betriebsspannung mit vorzugsweise 12 V, um den Motor mit einem Akku zu betreiben, zu der zweiten Betriebsspannung mit vorzugsweise 36 V, um den Motor über einen Netzanschluss zu betreiben, in einem Verhältnis von 1:N stehen. Es ist insbesondere bevorzugt, wenn jede Phasenspule drei Einzelspulen hat und die erste Betriebsspannung zur zweiten Betriebsspannung in einem Verhältnis von 1:3 stehen. Alternativ ist es bevorzugt, wenn jede Phasenspule zwei Einzelspulen hat und die erste Betriebsspannung zur zweiten Betriebsspannung in einem Verhältnis von 1:2 stehen. Ferner ist es bevorzugt, wenn jede Phasenspule vier oder mehr Einzelspulen hat und die erste Betriebsspannung zur zweiten Betriebsspannung in einem Verhältnis von 1:4, 1:5 usw. stehen.

Es ist von Vorteil, wenn das zumindest eine Kurzschlussmittel als ein passives Bauteil ausgebildet ist. Dadurch ist gegeben, dass durch die Montage eines einzigen passiven Bauteils die technische Umsetzung auf einfachste Art und Weise erfolgt. Des Weiteren hat der Einsatz eines solchen passiven Bauteils den Vorteil, dass dieses auch für den Einsatz in der Medizintechnik geeignet ist und entsprechenden Reinigungs-, Sterilisations- und Aufbereitungsprozeduren unterzogen werden kann.

Es ist ferner bevorzugt, wenn das zumindest eine Kurzschlussmittel auf einer ersten Einlegescheibe angebracht ist, mit welchem die N Einzelspulen derart kurzgeschlossen sind oder mit welchem Verbindungen zwischen den N Einzelspulen derart unterbrochen sind, dass die N Einzelspulen parallel miteinander verschalten sind, um den Motor in der ersten Betriebsspannung zu betreiben. Es ist verständlich, dass ein Vielfaches, vorzugsweise das Zweifache von der Anzahl an Einzelspulen an Kurzschlussmitteln auf der ersten Einlegescheibe angebracht ist. In anderen Worten bedeutet das, dass gemäß einer bevorzugten Ausführungsform beim Umschalten von der zweiten Betriebsspannung in die erste Betriebsspannung je Phase, also je drei Einzelspulen, zwei Kurzschlussmittel verwendet werden. Im Gegensatz dazu, wird gemäß einer bevorzugten Ausführungsform beim Antreiben mit der zweiten Betriebsspannung (bei Bedarf) je Phasenspule, also je drei Einzelspulen, nur ein Kurzschlussmittel verwendet.

Es ist vorteilhaft, wenn das zumindest eine Kurzschlussmittel auf einer zweiten Einlegescheibe angebracht ist, mit welchem Verbindungen zwischen den N Einzelspulen derart unterbrochen sind oder mit welchem die Einzelspulen derart kurzgeschlossen sind, dass die Einzelspulen in Reihe miteinander verschalten sind, um den Motor in der zweiten Betriebsspannung zu betreiben. Es ist verständlich, dass ein Vielfaches, vorzugsweise das Zweifache von der Anzahl an Einzelspulen an Kurzschlussmitteln auf der zweiten Einlegescheibe angebracht ist.

Des Weiteren ist es bevorzugt, wenn die erste Einlegescheibe so vorgesehen und ausgebildet ist, um zwischen den Motor und den Akku eingelegt zu sein. Dies hat den Vorteil, dass der Anwender lediglich die erste Einlegescheibe mechanisch einlegen muss, um von dem Netzbetrieb in den Akkubetrieb zu wechseln, das heißt von vorzugsweise 36 Volt auf 12 Volt Betriebsspannung umzuschalten. Somit muss zum Erreichen der jeweils niedrigeren Betriebsspannung die erste Einlegescheibe zwischengeschaltet werden. Dies kann jeweils durch den Anwender erfolgen oder die erste Einlegescheibe ist bereits in der niedrigeren Spannungsversorgung integriert sein. Ein Motor, der bislang nur im Netzbetrieb genutzt wurde, kann jetzt ohne weitere Modifikationen oder Manipulationen durch den Anwender im Akkubetrieb betrieben werden. Ein Netz (Kabel) Antrieb kann somit umgekehrt wahlweise auch mit Akku betrieben werden.

Es ist von Vorteil, wenn die zweite Einlegescheibe so vorgesehen und ausgebildet ist, um zwischen den Motor und den Netzanschluss eingelegt zu sein. Dies hat den Vorteil, dass der Anwender lediglich die zweite Einlegescheibe mechanisch einlegen muss, um von dem Akkubetrieb in den Netzbetrieb zu wechseln, das heißt von vorzugsweise 12 Volt auf 36 Volt Betriebsspannung umzuschalten. Somit muss zum Erreichen der jeweils höheren Betriebsspannung die zweite Einlegescheibe zwischengeschaltet werden. Dies kann jeweils durch den Anwender erfolgen oder die zweite Einlegescheibe ist bereits in der höheren Spannungsversorgung integriert sein. Ein Motor, der bislang nur im Akkubetrieb genutzt wurde, kann jetzt ohne weitere Modifikationen oder Manipulationen durch den Anwender im Netzbetrieb betrieben werden. Ein Akku Antrieb kann somit umgekehrt wahlweise auch mit Netz (Kabel) betrieben werden.

Es ist zu beachten, dass je nach Motorwicklungsverschaltung, welche aus den drei Einzelspulen besteht und welche in den elektrischen Motor integriert ist bzw. auf der Seite der Motorplattform angebracht ist, die dem elektrischen Motor zugewandt ist, in dem Netzbetrieb keine Einlegescheibe verwendet wird. In dem Fall, dass keine Einlegescheibe verwendet wird, sind die Einzelspulen bereits fest in Reihe miteinander verschaltet und mit dem Einlegen einer Einlegescheibe wird mittels der darauf befindlichen Kurzschlussmittel aus der Reihenschaltung eine Parallelschaltung und kann schließlich mit einem Akku betrieben werden.

Alternativ können die drei Einzelspulen auf der Motorplattform derart angebracht sein, dass für die Reihenschaltung bereits eine Einlegescheibe verwendet wird, welche die Einzelspulen entsprechend mittels einem Kurzschlussmittel verbindet. Diese Einlegescheibe wird beim Wechsel in den Akkubetrieb ausgetauscht.

Darüber hinaus kann es je nach konkreter konstruktiver Vorgabe verschiedener Motortypen nicht genügen, dass nur durch Kurzschließen der niedrigen Spannungsvariante die gewünschte Verschaltung erreicht wird. In diesem Fall müssen zusätzlich Verschaltungsverbindungen der höheren Betriebsspannung unterbrochen werden. Dies wird durch das Bereitstellen der zweiten Einlegescheibe für den Betrieb der höheren Betriebsspannungen bereitgestellt.

Es ist ferner vorteilhaft, wenn die Motorplattform je Phase vier Austrittsleiter hat, welche in Richtung des Akkus oder des Netzanschlusses gerichtet sind und in das Kurzschlussmittel einsteckbar sind.

Des Weiteren ist es von Vorteil, wenn die Verschaltung, die von der ersten Einlegescheibe oder der zweiten Einlegescheibe übernommen wird, elektronisch von der jeweils angeschlossenen Motorsteuerung aktiviert bzw. deaktiviert werden. Somit wäre ein händisches Einlegen bzw. Entfernen der ersten bzw. zweiten Einlegescheibe nicht mehr notwendig.

Es ist darüber hinaus bevorzugt, wenn die Motorplattform so vorgesehen und ausgebildet ist, dass die Motorcharakteristik und die Kennwerte des Motors beim Betrieb in den zwei zueinander unterschiedlichen ersten und zweiten Betriebsspannungen identisch sind. Das bedeutet, dass Parameter wie die Drehzahl oder das Drehmoment sowohl im Akkubetrieb als auch im Netzbetrieb identisch zueinander sind.

Darüber hinaus betrifft die vorliegende Erfindung eine Motorplattform, die eine gesamte Motorwicklungsverschaltung für das Betreiben eines elektrochirurgischen Instruments trägt, weist eine Schaltung auf, vorzugsweise eine Dreiecksschaltung oder Sternschaltung, die jeweils durch drei Phasen mit jeweils einer Phasenspule ausgebildet ist, und dabei zumindest eine Phasenspule aus N>1 miteinander verschalteten Einzelspulen ausgebildet ist, wobei in der ersten Betriebsspannung die N Einzelspulen jeder Phasenspule parallel und in der zweiten Betriebsspannung die N Einzelspulen jeder Phasenspule in Reihe geschaltet sind und das Umschalten zwischen der ersten und zweiten Betriebsspannung mittels zumindest eines Kurzschlussmittels je Phasenspule vorgesehen ist. Hierbei ist zu beachten, dass die Motorplattform mit den vorstehenden Aspekten kombinierbar ist.

Ferner betrifft die vorliegende Erfindung ein System mit einem elektrischen Motor und einer Motorwicklungsverschaltung gemäß einem der vorstehenden Aspekte, wobei der elektrische Motor dazu vorgesehen und ausgebildet ist, mit zwei unterschiedlichen Spannungsquellen betrieben zu werden.

Es ist bevorzugt, wenn die erste Spannungsquelle ein Energiespeicher ist, vorzugsweise ein Akku. Der Akku ist durch drei Batteriezellen ausgebildet, welche eine Spannung von 3 bis 4 Volt aufweisen, um den anzutreibenden Motor mit insgesamt $12\pm2$ Volt zu versorgen. Die Verwendung eines solchen Energiespeichers dient vorwiegend zum Betreiben von größeren chirurgischen Instrumenten. Die Verwendung von drei Batteriezellen hat den Vorteil, dass diese nicht zu groß bzw. zu schwer für den Einsatz in einem chirurgischen Instrument sind und zudem eine Spannung aufweisen, welche hoch genug ist, um den Motor mit ausreichender Leistung anzutreiben. Darüber hinaus ist die Zeit zwischen zwei Operationen/Anwendungen aufgrund der unabdingbaren Aufbereitung lang genug, um das Aufladen des Energiespeichers gewährleisten zu können. Aufgrund der Größe bzw. dem Gewicht ist eine Umsetzung mit einer höheren Spannung nicht zweckdienlich.

Es ist bevorzugt, wenn die zweite Spannungsquelle ein am Netz angeschlossenes Steuergerät ist, um den anzutreibenden Motor mit einer Spannung von 36 bis 39 Volt zu versorgen. Hierbei ist es vorteilhaft, dass die Spannungsversorgung nahe der 40 Volt ist, ab welcher die Normen für Luft- und Kriechströme erhöht werden würden. Eine Erhöhung der Luft- und Kriechströme und somit eine Spannung von 40 Volt oder mehr, würde gleichzeitig einen größeren Motor bedeuten, wodurch das chirurgische Instrument schwerer und unhandlicher werden würde. Dies ist insbesondere beim Betreiben von kleiner Knochenchirurgie, wie Hirn-/ Neurochirurgie etc., von Bedeutung, zu dessen Verwendung die zweite Spannungsquelle vorgesehen ist. Die vorstehend beschriebene erste Spannungsquelle ist für diesen Einsatz bereits zu groß und zu schwer und würde bei Verwendung im Sichtfeld des Bedieners sein. Ferner ist es bevorzugt, wenn die Spannung möglichst hoch ist, da die Ströme dann desto kleiner sind und somit dünnere Kabel verbaut werden können. Ein weiterer Vorteil der zweiten Spannungsquelle nahe der 40 Volt ist die regulatorische Freiheit unterhalb der 40 Volt größer als darüber. Das bedeutet, dass höhere Spannungen für Patienten gefährlicher sind und somit eine Spannung unterhalb der sogenannten "Schutzspannung", insbesondere bezüglich der Isolierung, vorteilhaft ist/ leichter umsetzbar ist.

Ferner ist es bevorzugt, wenn das vorstehend genannte System sterilisierbar ist. Daher ist eine ausreichende Isolation notwendig, welche bei Spannungen unterhalb der 40 Volt deutlich einfacher umgesetzt werden kann.

Zusammenfassend ist es Gegenstand der vorliegenden Erfindung, eine Motorwicklungsverschaltung vorzusehen, die den Betrieb eines Motors an zwei verschiedenen Betriebsspannungen ermöglicht. Die Motorcharakteristiken und die mechanischen Abgabewerte bleiben bei beiden Betriebsspannungen jedoch unverändert. Daher kann derselbe Motor bei gleicher operativer Anwendung wahlweise durch Netz oder Akku betrieben werden.

**Kurzbeschreibung der Figuren**

Fig. 1 ist eine Darstellung einer Motorwicklungsverschaltung in einer Dreiecksschaltung mit der zweiten Betriebsspannung;

Fig. 2 ist eine Darstellung einer Motorwicklungsverschaltung in einer Dreiecksschaltung mit der ersten Betriebsspannung;

Fig. 3 ist eine Darstellung einer Motorwicklungsverschaltung in einer Sternschaltung mit der zweiten Betriebsspannung;

Fig. 4 ist eine Darstellung einer Motorwicklungsverschaltung in einer Sternschaltung mit der ersten Betriebsspannung;

Fig. 5 ist eine vereinfachte Darstellung einer Verschaltung gemäß Fig. 4 mit zwei Kurzschlussmitteln 9 für den Antrieb mit einer ersten Betriebsspannung;

Fig. 6 ist eine vereinfachte Darstellung einer Verschaltung gemäß Fig. 3 mit zwei Kurzschlussmitteln 9 für den Antrieb mit einer zweiten Betriebsspannung;

Fig. 7 ist eine Explosionsdarstellung eines elektrischen Motors mit der ersten Betriebsspannung und der ersten Einlegescheibe;

Fig. 8 ist eine Explosionsdarstellung eines elektrischen Motors mit der zweiten Betriebsspannung und der zweiten Einlegescheibe;

Fig. 9 ist eine Darstellung des elektrischen Motors und dessen Motorplattform; und

Fig. 10 ist eine Darstellung der Motorwicklungsverschaltung der drei Einzelspulen einer Phasenspule im Netzbetrieb und im Akkubetrieb.

In den Figuren bezeichnen gleiche Bezugszeichen gleiche oder zumindest äquivalente Teile und Komponenten. Zweckmäßig wird insoweit eine mehrfache redundante Beschreibung solcher Teile und Komponenten weggelassen.

**Beschreibung bevorzugter Ausführungsformen**

Die Erfindung wird nachstehend anhand einer bevorzugten Ausführungsform unter Bezugnahme auf die begleitenden Figuren näher erläutert. Aufgrund der Übersichtlichkeit wurde in den Figuren nur eine Phasenspule 7 durch entsprechende Einzelspulen 8 ersetzt.

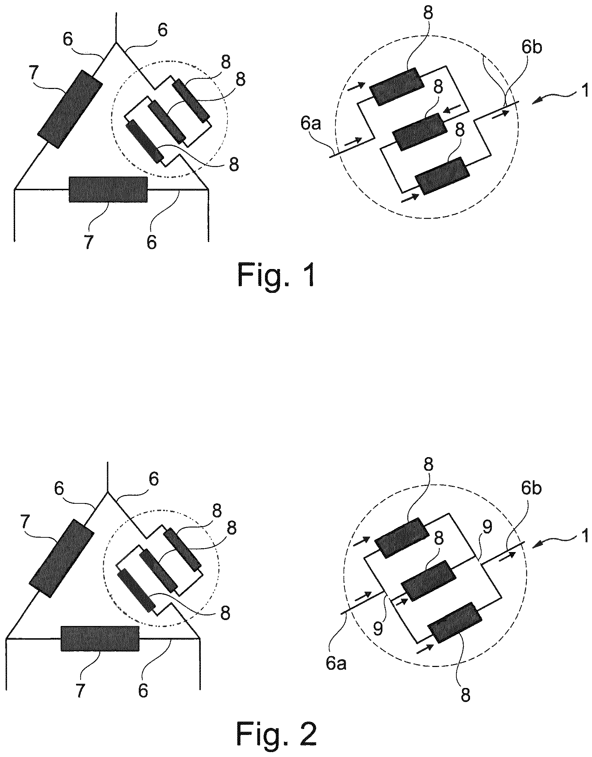

Fig. 1 ist eine Darstellung einer Motorwicklungsverschaltung 1 in einer Dreiecksschaltung mit der zweiten Betriebsspannung 5. Auf der linken Seite der Fig. 1 ist eine aus dem Stand der Technik bekannte Dreiecksschaltung zu sehen. Die Dreiecksschaltung besteht aus drei Phasen 6 mit jeweils einer Phasenspule 7. Gemäß einer bevorzugten Ausführungsform der vorliegenden Erfindung wird jede der drei Phasenspulen 7 durch drei Einzelspulen 8 ersetzt.

Auf der rechten Seite der Fig. 1 sind die drei Einzelspulen 8 zu erkennen. Die drei Einzelspulen 8 sind in Reihe zwischen einem Phaseneingang 6a und einem Phasenausgang 6b geschalten. Die dargestellten Pfeile auf der rechten Seite der Fig. 1 zeigen die Richtung des Stromflusses an.

Eine solche Motorverwicklungsschaltung 1 ist für den Netzbetrieb vorgesehen und weist die nachfolgende optimale Auslegung für die zweite Betriebsspannung 5 auf:

Tabelle 1

| Magnetfluss | Φ = konstant |
|---|---|
| Spannung | U (z.B. 36 Volt) |
| Windungszahl | N |
| Spulenstrom | I |

(fortgesetzt)

| Drahtquerschnitt | A |
|---|---|
| Widerstand | R |

Fig. 2 ist eine Darstellung einer Motorwicklungsverschaltung 1 als Dreiecksschaltung mit der ersten Betriebsspannung 4. Auf der linken Seite der Fig. 2 ist eine aus dem Stand der Technik bekannte Dreiecksschaltung zu sehen. Die Dreiecksschaltung besteht aus drei Phasen 6 mit jeweils einer Phasenspule 7. Gemäß einer bevorzugten Ausführungsform der vorliegenden Erfindung wird jede der drei Phasenspulen 7 durch drei Einzelspulen 8 ersetzt.

Die rechte Seite der Fig. 2 entspricht nahezu der rechten Seite der Fig. 1 mit dem Unterschied, dass zwei Kurzschlussmittel 9 die drei in Reihe geschalteten Einzelspulen 8 derart kurzschließen, so dass die drei Einzelspulen 8 nun parallel zwischen dem Phaseneingang 6a und dem Phasenausgang 6b verschaltet sind. Die dargestellten Pfeile auf der rechten Seite der Fig. 2 zeigen wiederum die Richtung des Stromflusses an.

Eine solche Motorverwicklungsschaltung 1 ist für den Akkubetrieb vorgesehen und weist die nachfolgende optimale Auslegung für die erste Betriebsspannung 4 auf:

Tabelle 2

| Magnetfluss | Φ = konstant |
|---|---|
| Spannung | U/3 (z.B. 12 Volt) |
| Windungszahl | N/3 |
| Spulenstrom | 3I |
| Drahtquerschnitt | 3A |
| Widerstand | R/3 |

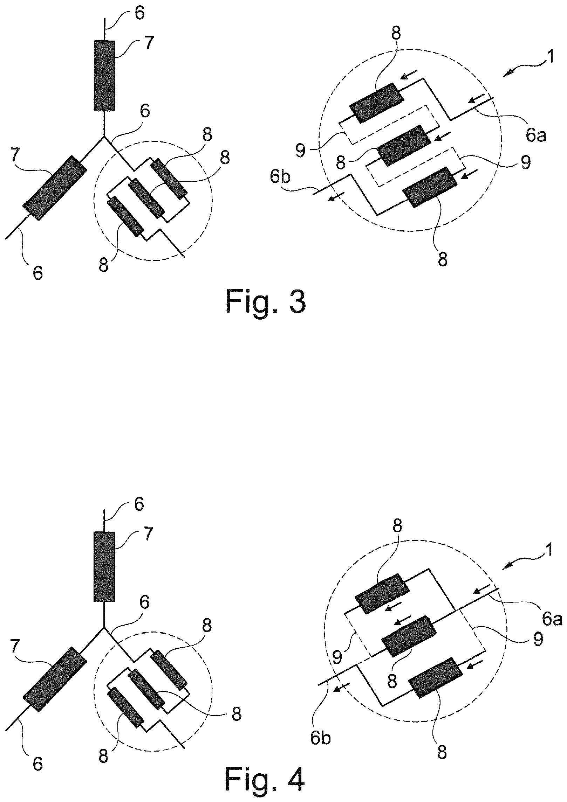

Fig. 3 ist eine Darstellung einer Motorwicklungsverschaltung 1 als Sternschaltung mit der zweiten Betriebsspannung 5. Auf der linken Seite der Fig. 3 ist eine aus dem Stand der Technik bekannte Sternschaltung zu sehen. Die Sternschaltung besteht aus drei Phasen 6 mit jeweils einer Phasenspule 7. Gemäß einer bevorzugten Ausführungsform der vorliegenden Erfindung wird jede der drei Phasenspulen 7 durch drei Einzelspulen 8, wie in Fig. 1, ersetzt.

Auf der rechten Seite der Fig. 3 sind die drei Einzelspulen 8 zu erkennen. Die drei Einzelspulen 8 sind zwischen dem Phaseneingang 6a und dem Phasenausgang 6b in Reihe geschalten. Die dargestellten Pfeile auf der rechten Seite der Fig. 3 zeigen die Richtung des Stromflusses an.

Eine solche Motorverwicklungsschaltung 1 ist für den Netzbetrieb vorgesehen und weist die gleiche optimale Auslegung für die zweite Betriebsspannung 5 gemäß vorstehender Tabelle 1 auf. Im Unterschied zu der Motorwicklungsverschaltung 1 gemäß Fig. 1 weist die Motorwicklungsverschaltung 1 gemäß Fig. 3 bereits zwei Kurzschlussmittel 9 auf, welche das Verschalten der drei Einzelspulen 8 zwischen dem Phaseneingang 6a und dem Phasenausgang 6b in Reihe ermöglichen.

Fig. 4 ist eine Darstellung einer Motorwicklungsverschaltung 1 als Sternschaltung mit der ersten Betriebsspannung 4. Auf der linken Seite der Fig. 4 ist eine aus dem Stand der Technik bekannte Sternschaltung zu sehen. Die Sternschaltung besteht aus drei Phasen 6 mit jeweils einer Phasenspule 7. Gemäß einer bevorzugten Ausführungsform der vorliegenden Erfindung wird jede der drei Phasenspulen 7 durch drei Einzelspulen 8 ersetzt.

Die rechte Seite der Fig. 4 entspricht nahezu der rechten Seite der Fig. 3 mit dem Unterschied, dass zwei Kurzschlussmittel 9 die drei in Reihe geschalteten Einzelspulen 8 derart kurzschließen, so dass die drei Einzelspulen 8 nun zwischen dem Phaseneingang 6a und dem Phasenausgang 6b parallel verschaltet sind. Die dargestellten Pfeile auf der rechten Seite der Fig. 4 zeigen wiederum die Richtung des Stromflusses an.

Eine solche Motorverwicklungsschaltung 1 ist für den Akkubetrieb vorgesehen und weist die gleiche optimale Auslegung für die erste Betriebsspannung 4 gemäß vorstehender Tabelle 2 auf. Im Unterschied zu der Motorwicklungsverschaltung 1 gemäß Fig. 2 weist die Motorwicklungsverschaltung 1 gemäß Fig. 4 andere Kurzschlussmittel 9 auf, welche das Verschalten der drei Einzelspulen 8 zwischen dem Phaseneingang 6a und dem Phasenausgang 6b parallel zueinander ermöglichen.

Aufgrund dieser vorstehenden Auslegungen und der Motorwicklungsverschaltungen 1 beim Betreiben des elektrischen Motors 2 in der ersten Betriebsspannung 4 gemäß Fign. 2 und 4 bzw. in der zweiten Betriebsspannung 5 gemäß Fign. 1 und 3, ist es möglich für die erste als auch die zweite Betriebsspannung 4 und 5 die gleiche Leistungscharakteristik zu erhalten. Daher gilt gemäß den nachstehenden Formeln folgender Zusammenhang zwischen der Reihenschaltung gemäß Fig. 1 bzw. 2 und der Parallelschaltung gemäß Fig. 2 bzw. 4:

$$U_{Reihe} = U_1 + U_2 + U_3 = 3U \ (\triangleq 36 \ \text{Volt})$$

$$U_{Parallel} = U_1 = U_2 = U_3 = U \ (\triangleq 12 \ \text{Volt})$$

Somit ist das Verhältnis von der ersten Betriebsspannung 4 zur zweiten Betriebsspannung 5 eins zu drei.

In einer alternativen Ausführungsform können lediglich zwei Einzelspulen 8 für jede Phasenspule 7 verwendet wird, mit dem Ergebnis, dass das Verhältnis von der ersten Betriebsspannung 4 zur zweiten Betriebsspannung 5 eins zu zwei ist. Dementsprechend können N>1 miteinander verschaltete Einzelspulen 8 verwendet werden, welche entsprechend das Verhältnis eins zu N ergeben.

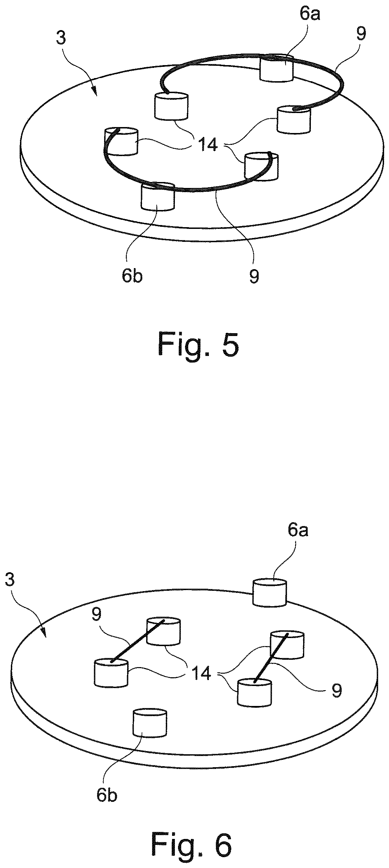

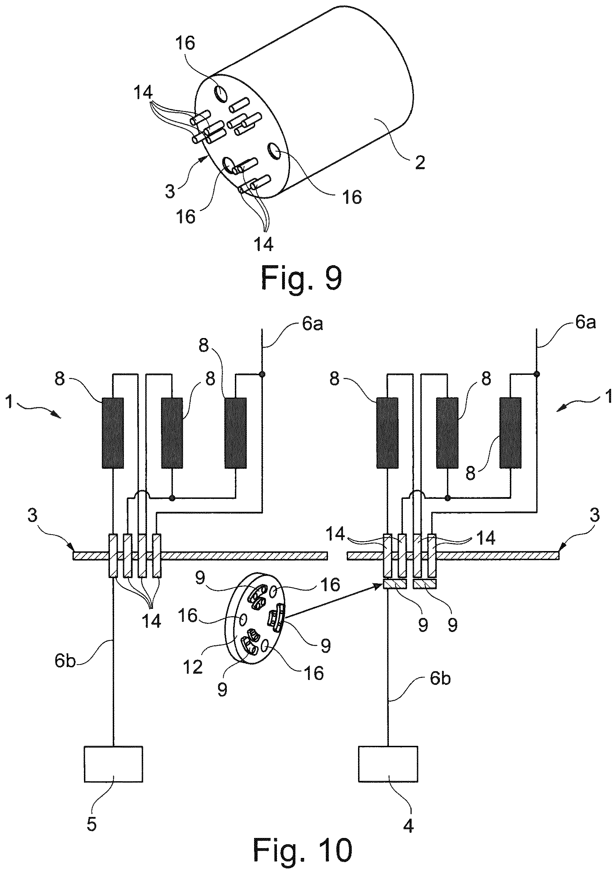

Fig. 5 ist eine vereinfachte Darstellung einer Verschaltung gemäß Fig. 4 mit zwei Kurzschlussmitteln 9 für den Antrieb mit einer ersten Betriebsspannung 4. Fig. 5 zeigt die Motorplattform 3, welche in Richtung hin zu dem Akku 10 oder dem Netzanschluss 11 gerichtet ist. Aus der Motorplattform 3 treten vier Austrittsleiter 14 heraus. Zwei der vier Austrittsleiter 14 sind mittels eines der beiden Kurzschlussmittel 9 miteinander kurzgeschlossen und mit dem Phaseneingang 6a verbunden. Die zwei weiteren der vier Austrittsleiter 14 sind mittels dem einen anderen Kurzschlussmittel 9 miteinander kurzgeschlossen und mit dem Phasenausgang 6b verbunden. Sämtliche Verbindungen der Austrittsleiter 14, welche auf der Motorplattform 3 nicht zu sehen sind, sind auf anderen Seite als feste Verbindungen vorgesehen, wie nachfolgend für eine bevorzugte Ausführungsform in Fig. 10 erläutert.

Fig. 6 ist eine vereinfachte Darstellung einer Verschaltung gemäß Fig. 3 mit zwei Kurzschlussmitteln 9 für den Antrieb mit einer zweiten Betriebsspannung 5. Fig. 6 zeigt die Motorplattform 3, welche in Richtung hin zu dem Akku 10 oder dem Netzanschluss 11 gerichtet ist. Aus der Motorplattform 3 treten vier Austrittsleiter 14 heraus. Zwei der vier Austrittsleiter 14 sind mittels eines der beiden Kurzschlussmittel 9 miteinander kurzgeschlossen. Die zwei weiteren der vier Austrittsleiter 14 sind mittels dem einen anderen Kurzschlussmittel 9 miteinander kurzgeschlossen. Sämtliche Verbindungen der Austrittsleiter 14, welche auf der Motorplattform 3 nicht zu sehen sind, sind auf anderen Seite als feste Verbindungen vorgesehen, wie nachfolgend für eine bevorzugte Ausführungsform in Fig. 10 erläutert.

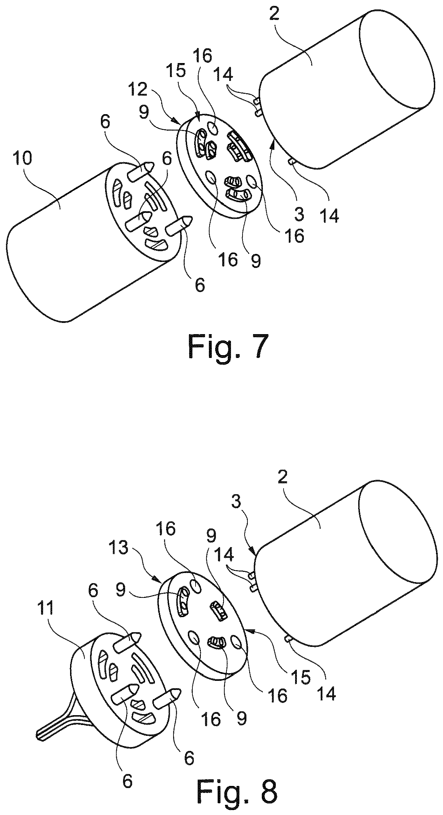

Fig. 7 ist eine Explosionsdarstellung eines elektrischen Motors 2 mit der ersten Betriebsspannung und der ersten Einlegescheibe 12. Fig. 7 zeigt den elektrischen Motor 2, den Akku 10 und die erste Einlegescheibe 12. Aus dem Akku 10 treten drei Phasen 6 heraus, welche den elektrischen Motor 2 mit Energie versorgen. Die erste Einlegescheibe 12 wird zwischen den elektrischen Motor 2 und den Akku 10 eingelegt. Die erste Einlegescheibe 12 hat drei Phasenlöcher 16. In jedes Phasenloch 16 wird eine aus dem Akku 10 herausstehende Phase 6 durchgesteckt. Auf der Motorauflageseite 15, welche die Seite ist, die von dem Akku 10 abgewendet ist, sind mehrere Kurzschlussmittel 9 angebracht, welche die Einzelspulen 8 aus den Figuren 2 und 4 miteinander kurzschließen.

Der elektrische Motor 2 weist ebenfalls drei Phasenlöcher 16 auf, wie in Fig. 9 gezeigt, in welche die Phasen 6 des Akkus 10 eingesteckt werden. Aus der Motorplattform 3 des elektrischen Motors 2 treten je Einzelspule 8, welche in dem elektrischen Motor 2 integriert sind, vier Austrittsleiter 14 heraus. Die jeweils vier Austrittsleiter 14 werden beim Einlegen der ersten Einlegescheibe 12 zwischen den elektrischen Motor 2 und den Akku 10 entsprechend kurzgeschlossen.

Fig. 8 ist eine Explosionsdarstellung eines elektrischen Motors 2 mit der zweiten Betriebsspannung und der zweiten Einlegescheibe 13. Fig. 8 zeigt den elektrischen Motor 2, den Netzanschluss 11 und die zweite Einlegescheibe 13. Aus dem Netzanschluss 11 treten drei Phasen 6 heraus, welche den elektrischen Motor 2 mit Energie versorgen. Die zweite Einlegescheibe 13 wird zwischen den elektrischen Motor 2 und den Netzanschluss 11 eingelegt. Die zweite Einlegescheibe 13 hast drei Phasenlöcher 16. In jedes Phasenloch 16 wird eine aus dem Netzanschluss 11 herausstehende Phase 6 durchgesteckt. Auf der Motorauflageseite 15, welche die Seite ist, die von dem Netzanschluss 11 abgewendet ist, sind mehrere Kurzschlussmittel 9 angebracht, welche die Einzelspulen 8 aus den Fign. 2 und 4 miteinander kurzschließen.

Der elektrische Motor 2 weist gemäß Fig. 7 ebenfalls drei Phasenlöcher 16 auf, wie in Fig. 9 gezeigt, in welche die Phasen 6 des Netzanschlusses 11 eingesteckt werden. Aus der Motorplattform 3 des elektrischen Motors 2 treten je Einzelspule 8, welche in dem elektrischen Motor 2 integriert sind, vier Austrittsleiter 14 heraus. Die jeweils vier Austrittsleiter 14 werden beim Einlegen der zweiten Einlegescheibe 13 zwischen den elektrischen Motor 2 und den Netzanschluss 11 entsprechend kurzgeschlossen.

Fig. 9 ist eine Darstellung des elektrischen Motors 2 und dessen Motorplattform 3. Aus der Motorplattform 3 treten je Einzelspule 8, die in den elektrischen Motor 2 integriert sind, vier Austrittsleiter 14 heraus. Diese werden mit Einlegen der ersten oder zweiten Einlegescheibe 12 oder 13 entsprechend der Fign. 5 und 6 mittels der Kurzschlussmittel 9 (nicht gezeigt) kurzgeschlossen. Somit kann diese Motorplattform unverändert in dem ersten Betriebsmodus 4 und in dem zweiten Betriebsmodus 5 verwendet werden.

Fig. 10 ist eine Darstellung der Motorwicklungsverschaltung 1 der drei Einzelspulen 8 einer Phasenspule 7 in dem elektrischen Motor 2 mit der ersten Betriebsspannung 4 und der zweiten Betriebsspannung 5. Fig. 10 zeigt auf der linken Seite die Motorwicklungsverschaltung 1 der drei Einzelspulen 8 und deren Verbindung zu den jeweiligen vier Austrittsleitern 14, die aus der Motorplattform 3 heraustreten, um den elektrischen Motor 2 mittels eines Netzanschlusses 11 in der zweiten Betriebsspannung 5 zu betreiben.

Die rechte Seite von Fig. 10 zeigt wiederum die Motorwicklungsverschaltung 1 gemäß der linken Seite und dessen vier Austrittsleiter 14, welche durch die Motorplattform 3 durchgesteckt sind. Jeweils zwei der vier Austrittsleiter 14 sind mittels eines Kurzschlussmittels 9 miteinander kurzgeschlossen, um den elektrischen Motor 2 mittels eines Akkus 10 in der ersten Betriebsspannung 4 zu betreiben.

Es ist zu beachten, dass die Motorplattform 3 als eine Platine ausgebildet sein kann, welche auf der dem elektrischen Motor 2 zugewandten Seite die Einzelspulen 8 und festen Verbindungen aufweist und auf der anderen Seite entsprechend die Austrittsleiter 14 austreten, welche mittels einer ersten oder zweiten Einlegescheibe 12 oder 13 kurzgeschlossen werden.

## Bezugszeichen

| 1 | Motorwicklungsverschaltung |
|---|---|
| 2 | Motor |
| 3 | Motorplattform |
| 4 | Erste Betriebsspannung |
| 5 | Zweite Betriebsspannung |
| 6 | Phase |
| 6a | Phaseneingang |
| 6b | Phasenausgang |
| 7 | Phasenspule |
| 8 | Einzelspule |
| 9 | Kurzschlussmittel |
| 10 | Akku |
| 11 | Netzanschluss |
| 12 | Erste Einlegescheibe |
| 13 | Zweite Einlegescheibe |
| 14 | Austrittsleiter |
| 15 | Motorauflageseite |
| 16 | Phasenloch |

## Patentansprüche

1. Motorplattform (3), die eine gesamte Motorwicklungsverschaltung (1) für das Betreiben eines elektrochirurgischen Instruments trägt, weist eine Schaltung auf, vorzugsweise eine Dreiecksschaltung oder Sternschaltung, die jeweils durch drei Phasen (6) mit jeweils einer Phasenspule (7) ausgebildet ist, und dabei zumindest eine Phasenspule (7) aus N>1 miteinander verschalteten Einzelspulen (8) ausgebildet ist, wobei die Ausgabe einer ersten Betriebsspannung (4) und einer zur ersten Betriebsspannung (4) unterschiedliche zweite Betriebsspannung (5) an einen Motor (2) vorgesehen ist, wobei in der ersten Betriebsspannung (4) die N Einzelspulen (8) jeder Phasenspule (7) parallel und in der zweiten Betriebsspannung (5) die N Einzelspulen (8) jeder Phasenspule (7) in Reihe geschaltet sind und das Umschalten zwischen der ersten und zweiten Betriebsspannung (4, 5) mittels zumindest eines Kurzschlussmittels (9) je Phasenspule (7) vorgesehen ist, wobei das zumindest eine Kurzschlussmittel (9) als ein passives Bauteil ausgebildet ist und wobei die Motorplattform (3) so vorgesehen und ausgebildet ist, dass die Motorcharakteristik und die Kennwerte des Motors (2) beim Betrieb in den zwei zueinander unterschiedlichen ersten und zweiten Betriebsspannungen (4, 5) identisch sind.

2. Elektrischer Motor (2) mit einer Motorwicklungsverschaltung (1) für das Betreiben eines elektrochirurgischen Instruments mit genau einer Motorplattform (3) gemäß Anspruch 1, die dazu vorgesehen und ausgebildet ist, den Motor (2) in einer ersten Betriebsspannung (4) und einer zur ersten Betriebsspannung (4) unterschiedlichen zweiten Betriebsspannung (5) zu betreiben und auf der Motorplattform (3) eine Schaltung vorgesehen ist, vorzugsweise eine Dreiecksschaltung oder Sternschaltung, die jeweils durch drei Phasen (6) mit jeweils einer Phasenspule (7) ausgebildet ist, und dabei zumindest eine Phasenspule (7) aus N>1 miteinander verschalteten Einzelspulen (8) ausgebildet ist.

3. Elektrischer Motor (2) gemäß Anspruch 2, wobei jede Phasenspule (7), vorzugsweise drei Einzelspulen (8) hat und die erste Betriebsspannung (4) mit vorzugsweise 12 V, um den Motor (2) mit einem Akku (10) zu betreiben, zu der zweiten Betriebsspannung (5) mit vorzugsweise 36 V, um den Motor (2) über einen Netzanschluss (11) zu betreiben, in einem Verhältnis von 1/N stehen.

4. Elektrischer Motor (2) gemäß Anspruch 2, wobei die erste Betriebsspannung (4) und die zweite Betriebsspannung (5) in einem Verhältnis von 1/3 stehen.

5. Elektrischer Motor (2) gemäß einem der Ansprüche 2 bis 4, wobei das zumindest eine Kurzschlussmittel (9) auf einer ersten Einlegescheibe (12) angebracht ist, mit welchem die N Einzelspulen (8) derart kurzgeschlossen sind oder mit welchem Verbindungen zwischen den N Einzelspulen (8) derart unterbrochen sind, dass die N Einzelspulen (8) parallel miteinander verschalten sind, um den Motor (2) in der ersten Betriebsspannung (4) zu betreiben.

6. Elektrischer Motor (2) gemäß einem der Ansprüche 2 bis 4, wobei das zumindest eine Kurzschlussmittel (9) auf einer zweiten Einlegescheibe (13) angebracht ist, mit welchem Verbindungen zwischen den N Einzelspulen (8) derart unterbrochen sind oder mit welchem die N Einzelspulen (8) derart kurzgeschlossen sind, dass die N Einzelspulen (8) in Reihe miteinander verschalten sind, um den Motor (2) in der zweiten Betriebsspannung (5) zu betreiben.

7. Elektrischer Motor (2) gemäß Anspruch 5 oder 6, wobei die erste Einlegescheibe (12) so vorgesehen und ausgebildet ist, um zwischen den Motor (2) und den Akku (10) eingelegt zu sein und/oder die zweite Einlegescheibe (13) so vorgesehen und ausgebildet ist, um zwischen den Motor (2) und den Netzanschluss (11) eingelegt zu sein.

8. Elektrischer Motor (2) gemäß einem der Ansprüche 2 bis 7, wobei die Motorplattform (3) je Phase (6) vier Austrittsleiter (14) hat, welche in Richtung des Akkus (10) oder des Netzanschlusses (11) gerichtet sind und in das Kurzschlussmittel (9) einsteckbar sind.

9. Chirurgisches Instrument mit einem elektrischen Motor (2) und einer Motorwicklungsverschaltung (1) gemäß einem der vorhergehenden Ansprüche.

10. System mit einem elektrischen Motor (2) und einer Motorwicklungsverschaltung (1) gemäß einem der Ansprüche 1 bis 9, wobei der elektrische Motor (2) dazu vorgesehen und ausgebildet ist, mit zwei unterschiedlichen Spannungsquellen betrieben zu werden.

**Claims**

1. A motor platform (3), which carries an entire motor winding interconnection (1) for operating an electrosurgical instrument, comprises a circuit, preferably a delta connection or star connection, which is formed by three phases (6) with one phase coil (7) each, and at least one phase coil (7) is formed from N>1 interconnected individual coils (8), wherein the output of a first operating voltage (4) and of a second operating voltage (5) different from the first operating voltage (4) is provided to a motor,
wherein, in the first operating voltage (4), the N individual coils (8) of each phase coil (7) are connected in parallel and in the second operating voltage (5), the N individual coils (8) of each phase coil (7) are connected in series, and the switching between the first and second operating voltages (4, 5) via at least one short-circuiting means (9) per phase coil (7) is provided, wherein the at least one short-circuiting means (9) is configured as a passive component and wherein the motor platform (3) is provided and designed in such a way that the motor characteristic and the characteristic values of the motor (2) are identical during operation in the first and second operating voltages (4, 5), which are different from each other.

2. The electric motor (2) with a motor winding interconnection (1) for operating an electrosurgical instrument with exactly one motor platform (3) according to claim 1, which is provided and configured to operate the motor (2) at a first operating voltage (4) and at a second operating voltage (5) different from the first operating voltage (4), and a circuit is provided on the motor platform (3), preferably a delta connection or star connection, which is formed in each case by three phases (6) with in each case one phase coil (7) each, and at least one phase coil (7) is formed from N>1 interconnected individual coils (8).

3. The electric motor (2) according to claim 2, wherein each phase coil (7) has preferably three individual coils (8) and the first operating voltage (4) with preferably 12 V for operating the motor (2) with a battery (10) is in a ratio of 1/N to the second operating voltage (5) with preferably 36 V for operating the motor (2) via a mains connection (11).

4. The electric motor (2) according to claim 1, wherein the first operating voltage (4) and the second operating voltage (5) are in a ratio of 1/3.

5. The electric motor (2) according to one of claims 2 to 4, wherein the at least one short-circuiting means (9) is mounted on a first insertion disk (12), with which the N individual coils (8) are short-circuited or with which connections between the N individual coils (8) are interrupted in such a way that the N individual coils (8) are connected in parallel with each other in order to operate the motor (2) at the first operating voltage (4).

6. The electric motor (2) according to one of claims 2 to 4, wherein the at least one short-circuiting means (9) is mounted on a second insertion disk (13), with which connections between the N individual coils (8) are interrupted or with which the N individual coils (8) are short-circuited in such a way that the N individual coils (8) are connected in series with each other in order to operate the motor (2) at the second operating voltage (5).

7. The electric motor (2) according to claim 5 or 6, wherein the first insertion disk (12) is provided and configured to be inserted between the motor (2) and the battery (10) and/or the second insertion disk (13) is provided and configured to be inserted between the motor (2) and the mains connection (11).

8. The electric motor (2) according to one of claims 2 to 7, wherein the motor platform (3) has four output conductors (14) per phase (6), which are directed towards the battery (10) or the mains connection (11) and can be plugged into the short-circuiting means (9).

9. A surgical instrument comprising an electric motor (2) and a motor winding interconnection (1) according to one of the preceding claims.

10. A system comprising an electric motor (2) and a motor winding interconnection (1) according to one of claims 1 to 9, wherein the electric motor (2) is provided and adapted to be driven by two different voltage sources.

**Revendications**

1. Plateforme de moteur (3) qui porte un circuit d'enroulement de moteur (1) complet pour le fonctionnement d'un instrument électrochirurgical, présente un circuit, de préférence un circuit en triangle ou un circuit en étoile, qui est formé respectivement par trois phases (6) avec respectivement une bobine de phase (7), et au moins une bobine de phase (7) est formée à cet effet de N>1 bobines individuelles (8) interconnectées, dans laquelle la sortie d'une première tension de fonctionnement (4) et d'une seconde tension de fonctionnement (5) différente de la première tension de fonctionnement (4) est prévue au niveau d'un moteur (2),
dans laquelle les N bobines individuelles (8) de chaque bobine de phase (7) sont montées en parallèle dans la première tension de fonctionnement (4) et les N bobines individuelles (8) de chaque bobine de phase (7) sont montées en série dans la seconde tension de fonctionnement (5), et la commutation entre la première et la seconde tension de fonctionnement (4, 5) est prévue au moyen d'au moins un moyen de court-circuitage (9) par bobine de phase (7), dans laquelle le au moins un moyen de court-circuitage (9) est conçu comme un composant passif et dans laquelle la plateforme de moteur (3) est prévue et conçue de sorte que la caractéristique de moteur et les valeurs caractéristiques du moteur (2) soient identiques lors du fonctionnement dans les deux première et seconde tensions de fonctionnement (4, 5) différentes l'une de l'autre.

2. Moteur électrique (2) avec un circuit d'enroulement de moteur (1) pour le fonctionnement d'un instrument électrochirurgical avec exactement une plateforme de moteur (3) selon la revendication 1,
qui est prévu et conçu pour faire fonctionner le moteur (2) dans une première tension de fonctionnement (4) et une seconde tension de fonctionnement (5) différente de la première tension de fonctionnement (4), et un circuit est prévu sur la plateforme de moteur (3), de préférence un circuit en triangle ou un circuit en étoile, qui est formé respectivement par trois phases (6) avec respectivement une bobine de phase (7), et au moins une bobine de phase (7) est formée à cet effet de N>1 bobines individuelles (8) interconnectées.

3. Moteur électrique (2) selon la revendication 2, dans lequel chaque bobine de phase (7) présente de préférence trois bobines individuelles (8) et la première tension de fonctionnement (4), de préférence de 12 V pour faire fonctionner le moteur (2) avec un accumulateur (10), est dans un rapport de 1/N par rapport à la seconde tension de fonctionnement (5) de préférence de 36 V pour faire fonctionner le moteur (2) par l'intermédiaire d'un raccordement au réseau (11).

4. Moteur électrique (2) selon la revendication 2, dans lequel la première tension de fonctionnement (4) et la seconde tension de fonctionnement (5) sont dans un rapport de 1/3.

5. Moteur électrique (2) selon l'une quelconque des revendications 2 à 4, dans lequel le au moins un moyen de court-circuitage (9) est placé sur un premier disque d'insertion (12) avec lequel les N bobines individuelles (8) sont court-circuitées ou avec lequel des connexions entre les N bobines individuelles (8) sont interrompues de sorte que les N bobines individuelles (8) soient interconnectées en parallèle pour faire fonctionner le moteur (2) dans la première tension de fonctionnement (4).

6. Moteur électrique (2) selon l'une quelconque des revendications 2 à 4, dans lequel le au moins un moyen de court-circuitage (9) est placé sur un second disque d'insertion (13) avec lequel des connexions entre les N bobines individuelles (8) sont interrompues ou avec lequel les N bobines individuelles (8) sont court-circuitées de sorte que les N bobines individuelles (8) soient interconnectées en série pour faire fonctionner le moteur (2) dans la seconde tension de fonctionnement (5).

7. Moteur électrique (2) selon la revendication 5 ou 6, dans lequel le premier disque d'insertion (12) est prévu et conçu pour être inséré entre le moteur (2) et l'accumulateur (10) et/ou le second disque d'insertion (13) est prévu et conçu pour être inséré entre le moteur (2) et le raccordement au réseau (11).

8. Moteur électrique (2) selon l'une quelconque des revendications 2 à 7, dans lequel la plateforme de moteur (3) présente quatre conducteurs de sortie (14) par phase (6) qui sont dirigés vers l'accumulateur (10) ou le raccordement au réseau (11) et peuvent être insérés dans le moyen de court-circuitage (9).

9. Instrument chirurgical avec un moteur électrique (2) et un circuit d'enroulement de moteur (1) selon l'une quelconque des revendications précédentes.

10. Système avec un moteur électrique (2) et un circuit d'enroulement de moteur (1) selon l'une quelconque des revendications 1 à 9, dans lequel le moteur électrique (2) est prévu et conçu pour fonctionner avec deux sources de tension différentes.

Fig. 1

Fig. 2

Fig. 3

Fig. 4

Fig. 5

Fig. 6

Fig. 7

Fig. 8

Fig. 9

Fig. 10

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102013009036 A1
- EP 3400645 A1
- JP 2017121158 A
- EP 3435540 A1
- US 2018256287 A1
- WO 2013155601 A1
- EP 1450469 A2
- WO 2018213919 A1
- EP 3534532 A1